# DEMANDE DE BREVET EUROPEEN

(11) **EP 1 719 477 A1**
(43) Date de publication de la demande: **08.11.2006**
(21) Numéro de dépôt: 06356004.9
(22) Date de dépôt: 10.01.2006
(51) Int. Cl.: A61F 2/30

(54) **Prothese d'epaule**

(30) Priorité: 20.01.2005 FR 0500564
(71) Demandeur: Groupe Lepine, 69394 Lyon Cedex 03 (FR)
(72) Inventeur: Chirpaz- Cerbat, Jean Marie,, 74000 Annecy (FR); Martinez, Thierry, 73310 Serrieres en Chautagne (FR); Saragaglia, Dominique, 38640 Claix (FR)
(74) Mandataire: Jeannet, Olivier

(57) **Abrégé**

Cette prothèse d'épaule (1) inclut une tête prothétique (2) et des moyens d'ancrage de cette tête prothétique (2) à l'humérus, en particulier une tige médullaire (3).

Selon l'invention, la prothèse (1) comprend au moins une partie (11) faisant saillie sur le côté externe de la tête prothétique (2), au niveau de la base de cette tête, cette ou ces parties en saillie (11) étant conformées pour prendre appui contre les tubérosités ou contre les tissus environnant ces tubérosités de manière à empêcher toute migration proximale de ces dernières.

## Description

La présente invention concerne une prothèse d'épaule.

Une fracture de l'articulation de l'épaule peut conduire, particulièrement en cas d'ostéoporose, à une rupture de la partie supérieure de l'humérus en trois ou quatre fragments, à savoir la tête humérale, les tubérosités, c'est-à-dire le trochin et le trochiter, et la diaphyse. Les deux tubérosités peuvent elles-mêmes être séparées l'une de l'autre.

Une technique classique de traitement d'une fracture de l'épaule consiste à mettre en place une prothèse d'épaule comprenant une tête humérale prothétique, avec positionnement d'un greffon osseux sous cette tête prothétique, du côté externe, puis à opérer une synthèse des tubérosités et de l'humérus, en maintenant les tubérosités contre ce greffon.

La croissance des cellules osseuses permet de ressouder les tubérosités et l'humérus. Il arrive cependant que la soudure osseuse ne s'opère pas de manière optimale, ou que le patient entreprenne une rééducation trop précoce, alors que la consolidation osseuse n'est pas encore suffisante. Compte tenu des efforts exercés sur les tubérosités par les muscles reliés à elles, le risque existe alors que se produise une migration proximale des tubérosités. Cette migration entraîne un conflit des tubérosités avec l'acromion, empêchant ultérieurement le patient de lever son bras au-dessus de l'épaule.

Les documents US 6,171,341 et WO 93/09733 décrivent des prothèses d'épaule ne donnant pas parfaitement satisfaction pour le traitement de fractures intervenant au niveau des tubérosités d'une épaule, n'étant pas conformée de manière à empêcher toute migration proximale des tubérosités.

La présente invention vise à remédier à cet inconvénient essentiel.

Son objectif est de fournir une prothèse d'épaule permettant de supprimer tout risque d'une migration proximale des tubérosités.

La prothèse d'épaule concernée inclut, de manière connue en soi, une tête prothétique et des moyens d'ancrage de cette tête prothétique à l'humérus, en particulier une tige médullaire.

Selon l'invention, la prothèse comprend au moins une partie faisant saillie sur le côté externe de la tête prothétique, au niveau de la base de cette tête, cette ou ces parties en saillie étant conformées pour prendre appui contre les tubérosités ou contre les tissus environnant ces tubérosités de manière à empêcher toute migration proximale de ces dernières.

Tout risque de migration des tubérosités est ainsi évité de manière efficace.

De préférence, la ou lesdites parties en saillie s'étendent sur un arc important de la base de la tête prothétique, de part et d'autre du plan intéro-externe médian de la prothèse. Cet arc peut notamment être de l'ordre d'une centaine de degrés.

La ou lesdites parties en saillie permettent ainsi une prise d'appui répartie contre les tubérosités ou les tissus environnant ces dernières.

Cette ou ces parties en saillie peuvent être pointues au niveau de leurs extrémités libres, afin de pouvoir être légèrement insérées dans les tissus environnant les tubérosités, en particulier dans la base des tendons reliés au tubérosités.

La prothèse peut comprendre une seule partie en saillie, sous forme d'un rebord. Selon une forme de réalisation préférée de l'invention, toutefois, la prothèse comprend une pluralité de parties en saillie sous forme de dents, espacées les unes des autres.

Les dents peuvent avoir des longueurs constantes mais ont de préférence des longueurs non égales, plus importantes au niveau dudit plan intéro-externe médian de la prothèse et allant en se raccourcissant, d'une dent à l'autre, en s'éloignant de ce plan.

Ces mêmes dents peuvent former des angles constants avec la surface définissant la base de la tête prothétique mais forment de préférence des angles non constants avec cette surface, les dents situées au niveau dudit plan intéro-externe médian de la prothèse formant des angles relativement ouverts, de l'ordre de 135°, avec ladite surface définissant la base de la tête prothétique, et ces mêmes angles se refermant progressivement, d'une dent à l'autre, en s'éloignant dudit plan intéro-externe médian de la prothèse, jusqu'à des valeurs de l'ordre de 100°, pour les dents les plus éloignées dudit plan intéro-externe médian.

Les dents peuvent être sécables ou déformables au moins au niveau de leurs bases. Certaines dents peuvent ainsi être supprimées si nécessaire, ou être orientées en fonction des besoins.

La ou lesdites parties saillantes sont avantageusement solidaires d'une partie de base destinée à être montée contre la tête prothétique, cette ou ces parties saillantes et cette partie de base formant une pièce d'appui indépendante de la tête prothétique.

Cette pièce d'appui peut être fixée ou non contre la tête prothétique selon le type de fracture à traiter.

La prothèse selon l'invention comprend avantageusement une tête prothétique séparée desdits moyens d'ancrage à l'humérus et pouvant être assemblée à ceux-ci selon plusieurs positions angulaires possibles, afin de permettre un réglage du déport médial et postérieur de l'articulation. Dans ce cas, les moyens de fixation de ladite pièce d'appui à la tête prothétique sont avantageusement prévus pour permettre un montage de la pièce d'appui selon plusieurs positions angulaires possibles par rapport à la tête prothétique, de telle sorte que ladite pièce d'appui puisse toujours être disposée de manière substantiellement symétrique par rapport audit plan intéro-externe médian de la prothèse quelle que soit la position angulaire de montage de la tête prothétique par rapport auxdits moyens d'ancrage.

Selon une forme de réalisation préférée de l'invention dans ce cas, la partie de base de la pièce d'appui comprend au moins deux trous de réception de vis permettant son montage sur la tête prothétique, et cette dernière comprend un nombre de trous supérieur à deux, disposés sur un cercle coaxial à la tête prothétique, permettant de fixer ladite pièce d'appui selon au moins deux positions par rapport à cette tête prothétique. La pièce d'appui peut notamment comprendre trois trous de montage distant angulairement de 45 degrés, et la tête prothétique peut comprendre sept trous correspondants.

De préférence, la tête prothétique présente un bossage central coaxial à elle, contre lequel ladite pièce d'appui vient s'appliquer lors de son montage sur cette tête prothétique, ce bossage permettant de faciliter le positionnement radial de ladite pièce d'appui par rapport à la tête prothétique.

La venue en coïncidence des trous de la pièce d'appui et des trous de la tête prothétique peut ainsi être réalisée de manière facilitée.

Pour sa bonne compréhension, l'invention est à nouveau décrite ci-dessous, en référence au dessin schématique annexé représentant, à titre d'exemple non limitatif, une forme de réalisation préférée de la prothèse d'épaule qu'elle concerne.
La figure 1 en est une vue en perspective éclatée ;
les figures 2 à 6 sont des vues d'une pièce d'appui qu'elle comprend, respectivement en perspective sous un premier angle, en perspective sous un autre angle, de face, de côté et de profil ;
la figure 7 est une vue de la prothèse similaire à la figure 1, après assemblage ;
la figure 8 est une vue de la prothèse dans le plan intéro-externe ;
la figure 9 est une vue de la prothèse selon l'axe d'une tête prothétique que comprend cette prothèse, dans une autre position de montage de cette tête prothétique sur une tige médullaire que comprend également la prothèse, et
la figure 10 est une vue de la prothèse similaire à la figure 9, selon encore une autre position de montage de la tête prothétique sur la tige médullaire.

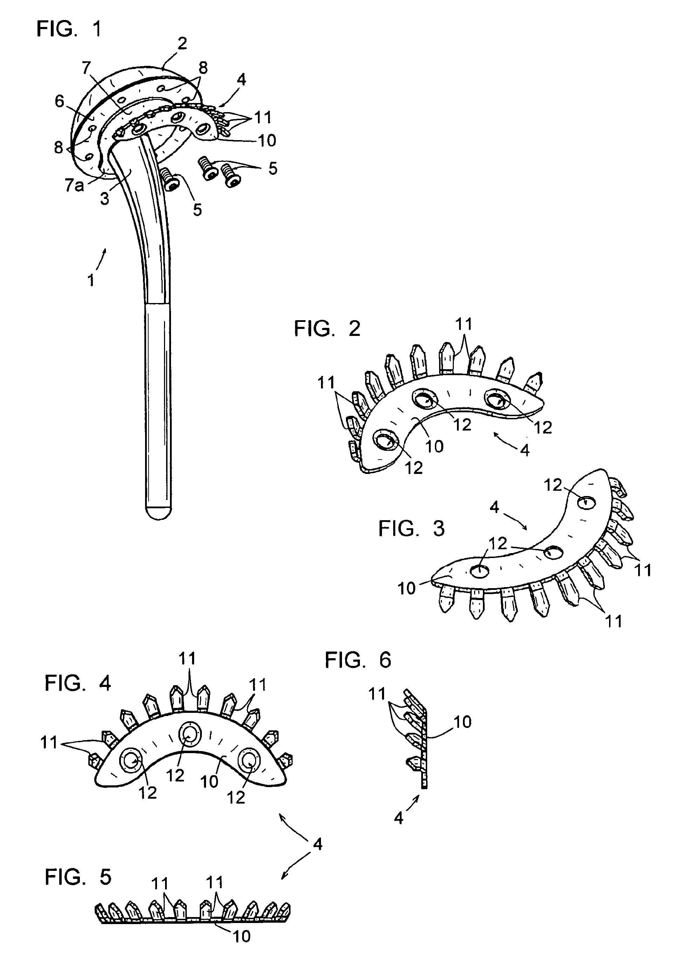

La figure 1 représente une prothèse d'épaule 1, comprenant une tête prothétique 2, une tige médullaire 3, une pièce d'appui 4 et des vis 5 de montage de cette pièce d'appui 4 sur la tête prothétique 2.

La tête prothétique 2 présente une forme en calotte sphérique dont la base est limitée par une paroi 6. Au niveau de cette paroi 6, la tête 2 présente une cavité octogonale décalée par rapport à son axe, et comprend un bossage central 7 ainsi que sept trous périphériques 8.

La cavité octogonale est destinée à recevoir un plot octogonal aménagé sur l'extrémité proximale de la tige médullaire 3, ce plot ayant la même forme que cette cavité mais ayant des dimensions légèrement très inférieures à celle-ci, de sorte qu'il peut être reçu de manière ajustée dans cette cavité. La cavité et le plot ont par ailleurs des formes allant en se rétrécissant progressivement en direction du fond de la cavité, de sorte qu'un coincement du plot dans la cavité soit possible et permette d'assurer l'assemblage résistant de la tête 2 à la tige médullaire 3.

Grâce à la forme octogonale de cette cavité et de ce plot, la tête 2 peut être assemblée à la tige médullaire 3 selon plusieurs positions angulaires possibles, décalées de 45 degrés, ainsi que cela apparaît par comparaison des figures 1, 9 et 10, afin de permettre un réglage du déport médial et postérieur de l'articulation prothétique.

Le bossage 7 est coaxial à la tête 2, et, en dehors d'une extension radiale 7a qu'il forme sur un secteur de la périphérie de la tête 2, est circulaire.

Les trous 8 sont régulièrement répartis sur un cercle coaxial à la tête 2, en étant disposés à 45 degrés les uns des autres. Ces trous 8 sont aménagés sensiblement à mi-distance entre la périphérie de la tête 2 et la périphérie du bossage 7 et sont destinés à recevoir les vis 5.

La tige médullaire 3 présente une partie métaphysaire courbe et une partie diaphysaire rectiligne, adaptées à une introduction dans le canal médullaire de l'humérus.

La pièce d'appui 4 comprend une partie de base 10 destinée à être montée contre la tête 2 et une série de dents 11 solidaires de cette partie de base 10 et faisant saillie de celle-ci.

Comme le montrent plus particulièrement les figures 2 à 6, la partie de base 10 présente une forme en arc de cercle, et a un bord interne défini par un rayon légèrement supérieur à celui du bossage 7 est un bord externe défini par un rayon légèrement inférieur à celui de la tête 2. Cette partie de base 10 s'étend sur un arc d'environ 100 degrés.

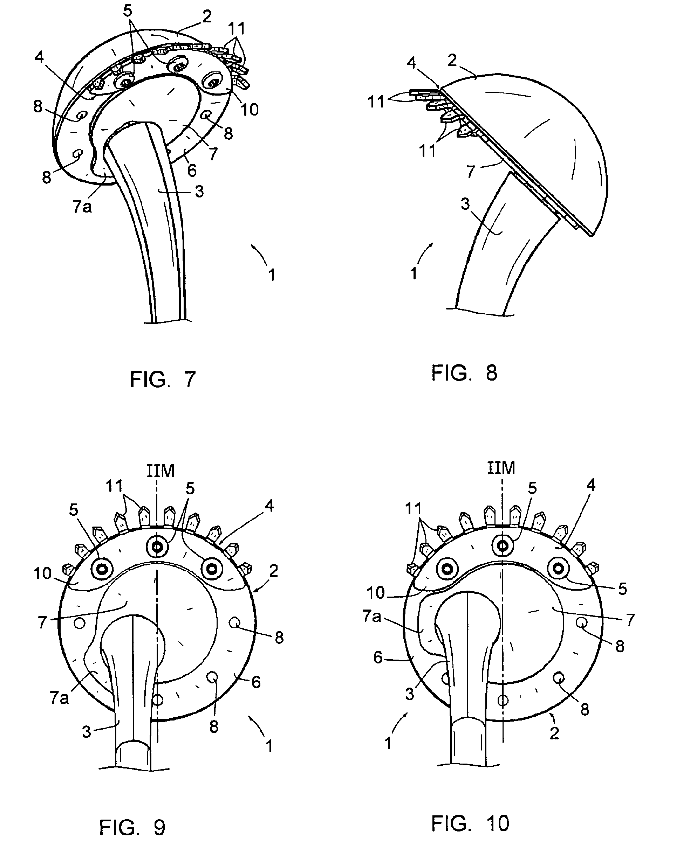

La partie de base 10 comprend par ailleurs trois trous 12 pouvant être traversés par les corps filetés des vis 5, les têtes de ces vis 5 étant reçues dans des fraisages aménagés sur la face de la partie de base 10 visible sur les figures 2 et 4. Ces trous 12 sont aménagés à des distances angulaires de 45 degrés et peuvent ainsi venir en regard des trous 8 pour permettre la fixation de la pièce 4 à la tête 2 selon plusieurs positions différentes, ainsi que cela apparaît par comparaison des figures 7, 9 et 10.

Les dents 11 font saillie de bord externe de la partie de base 10. Elles sont espacées les unes des autres, ont des longueurs non égales et forment des angles non constants avec la partie de base 10: les longueurs des dents 11 sont plus importantes au niveau du plan de symétrie de la pièce 4 et vont en se raccourcissant, d'une dent 11 à l'autre, en s'éloignant de ce plan ; les dents 11 situées au niveau du plan de symétrie forment des angles de l'ordre de 135° avec la partie de base 10 (cf. figure 6), et ces mêmes angles se referment progressivement, d'une dent 11 à l'autre, en s'éloignant du plan de symétrie, jusqu'à des valeurs de l'ordre de 100°, pour les dents 11 les plus éloignées de ce plan de symétrie.

Les extrémités libres des dents 11 sont taillées en pointe, de sorte que ces dents peuvent être légèrement insérées dans les tissus environnant les tubérosités, en particulier dans la base des tendons reliés aux tubérosités.

Comme le montrent les figures 7, 9 et 10, les trous 12 et 8 et les vis 5 permettent un montage de la pièce d'appui 4 de telle sorte que cette pièce d'appui puisse toujours être disposée de manière substantiellement symétrique par rapport au plan intéro-externe médian IIM de la prothèse 1, quelle que soit la position angulaire de montage de la tête 2 par rapport à la tige 3.

Lors de son montage, la pièce d'appui 4 vient s'appliquer contre le bossage 7, ce qui permet de faciliter son positionnement radial par rapport à la tête 2 et donc la venue en coïncidence des trous 12 et des trous 8.

La figure 8 montre qu'après montage de la pièce 4 sur la tête 2, les dents 11 font saillie sur le côté externe de la tête 2, au niveau de la base de cette tête, et sont ainsi à même de prendre appui contre les tubérosités ou contre les tissus environnant ces tubérosités.

Comme cela apparaît de ce qui précède, l'invention fournit une prothèse d'épaule ayant pour avantage déterminant de permettre de prévenir efficacement tout risque d'une migration proximale des tubérosités, particulièrement lorsque la soudure osseuse ne s'opère pas de manière optimale, ou que le patient entreprend une rééducation précoce.

Il va de soi que l'invention n'est pas limitée à la forme de réalisation de l'invention décrite ci-dessus à titre d'exemple mais qu'elle s'étend à toutes les variantes de réalisation couverte par les revendications ci-annexées.

## Revendications

1. Prothèse d'épaule (1), incluant une tête prothétique (2) et des moyens d'ancrage de cette tête prothétique (2) à l'humérus, en particulier une tige médullaire (3), **caractérisée en ce qu'**elle comprend au moins une partie (11) faisant saillie sur le côté externe de la tête prothétique (2), au niveau de la base de cette tête, cette ou ces parties en saillie (11) étant conformées pour prendre appui contre les tubérosités ou contre les tissus environnant ces tubérosités de manière à empêcher toute migration proximale de ces dernières.

2. Prothèse d'épaule (1) selon la revendication 1, **caractérisée en ce que** la ou lesdites parties en saillie (11) s'étendent sur un arc important de la base de la tête prothétique (2), de part et d'autre du plan intéro-externe médian de la prothèse.

3. Prothèse d'épaule (1) selon la revendication 1 ou la revendication 2, **caractérisée en ce que** la ou lesdites parties en saillie (11) sont pointues au niveau de leurs extrémités libres, afin de pouvoir être légèrement insérées dans les tissus environnant les tubérosités, en particulier dans la base des tendons reliés au tubérosités.

4. Prothèse d'épaule (1) selon l'une des revendications 1 à 3, **caractérisée en ce qu'**elle comprend une pluralité de parties en saillie (11) espacées les unes des autres, sous forme de dents.

5. Prothèse d'épaule (1) selon la revendication 4, **caractérisée en ce que** les dents (11) ont des longueurs non égales, plus importantes au niveau du plan intéro-externe médian de la prothèse et allant en se raccourcissant, d'une dent (11) à l'autre, en s'éloignant de ce plan.

6. Prothèse d'épaule (1) selon la revendication 4 ou la revendication 5, **caractérisée en ce que** les dents (11) forment des angles non constants avec la surface (6) définissant la base de la tête prothétique (2), les dents (11) situées au niveau du plan intéro-externe médian de la prothèse formant des angles relativement ouverts, de l'ordre de 135°, avec ladite surface (6) définissant la base de la tête prothétique (2), et ces mêmes angles se refermant progressivement, d'une dent (11) à l'autre, en s'éloignant du plan intéro-externe médian de la prothèse, jusqu'à des valeurs de l'ordre de 100°, pour les dents (11) les plus éloignées du plan intéro-externe médian.

7. Prothèse d'épaule (1) selon l'une des revendications 1 à 6, **caractérisée en ce que** la ou lesdites parties saillantes (11) sont solidaires d'une partie de base (10) destinée à être montée contre la tête prothétique (2), cette ou ces parties saillantes (11) et cette partie de base (10) formant une pièce d'appui (4) indépendante de la tête prothétique (2).

8. Prothèse d'épaule (1) selon la revendication 7, **caractérisée en ce qu'**elle comprend une tête prothétique (2) séparée desdits moyens (3) d'ancrage à l'humérus et pouvant être assemblée à ceux-ci selon plusieurs positions angulaires possibles, afin de permettre un réglage du déport médial et postérieur de l'articulation, et **en ce que** les moyens (5, 8, 12) de fixation de ladite pièce d'appui (4) à la tête prothétique (2) sont prévus pour permettre un montage de la pièce d'appui (4) selon plusieurs positions angulaires possibles par rapport à la tête prothétique (2), de telle sorte que ladite pièce d'appui (4) puisse être disposée de manière substantiellement symétrique par rapport au plan intéro-externe médian de la prothèse (1) quelle que soit la position angulaire de montage de la tête prothétique (2) par rapport auxdits moyens d'ancrage (3).

9. Prothèse d'épaule (1) selon la revendication 8, **caractérisée en ce que** la partie de base (10) de la pièce d'appui (4) comprend au moins deux trous (12) de réception de vis (5) permettant son montage sur la tête prothétique (2), et **en ce que** cette dernière comprend un nombre de trous (8) supérieur à deux, disposés sur un cercle coaxial à la tête prothétique (2), permettant de fixer ladite pièce d'appui (4) selon au moins deux positions par rapport à cette tête prothétique (2).

10. Prothèse d'épaule (1) selon la revendication 8 ou la revendication 9, **caractérisée en ce que** la tête prothétique (2) présente un bossage central (7) coaxial à elle, contre lequel ladite pièce d'appui (4) vient s'appliquer lors de son montage sur cette tête prothétique (2).
